# EUROPEAN PATENT APPLICATION

(11) **EP 4 144 752 A1**
(43) Date of publication of application: **08.03.2023**
(21) Application number: 21306199.7
(22) Date of filing: 02.09.2021
(51) Int. Cl.: C07K 14/005, A61K 39/12, C07K 14/47, C07K 14/44

(54) **VIRAL-LIKE PARTICLES FOR THE TREATMENT OR PREVENTION OF AN INFECTION BY A CORONAVIRIDAE VIRUS**

(71) Applicant: SORBONNE UNIVERSITE, 75006 Paris (FR); INSTITUT NATIONAL DE LA SANTE ET DE LA RECHERCHE MEDICALE - INSERM, 75013 Paris (FR); ASSISTANCE PUBLIQUE, HOPITAUX DE PARIS, 75004 Paris (FR); Consejo Nacional de Investigaciones Científicas Y Técnicas (CONICET), Caba 1425 (AR); Universidad Católica de Córdoba (UCC), Ciudad de Córdoba 5000 (AR)
(72) Inventor: Klatzmann, David, 75013 PARIS (FR); Lujan, Hugo, 5009 Córdoba (AR)
(74) Representative: Gevers & Orès

(57) **Abstract**

The invention pertains to new viral-like particles (VLPs), pharmaceutical compositions comprising the same and methods of using the same to prevent or treat an infection by a *Coronaviridae* virus. Advantageously, these VLPs can be used as a vaccine to be orally or nasally administrated.

## Description

The invention pertains to new viral-like particles (VLPs), pharmaceutical compositions comprising the same and methods of using the same to prevent or treat an infection by a *Coronaviridae* virus. Advantageously, these VLPs can be used as a vaccine to be orally or nasally administrated.

Before 2019, only six coronaviruses - Human Coronavirus 229E (HCoV-229E), Human Coronavirus OC43 (HCoV-OC43), Severe Acute Respiratory Syndrome Coronavirus (SARS-CoV), Human Coronavirus NL63 (HCoV-NL63), Human Coronavirus HKU1 (HCoV-HKU1), and Middle East Respiratory Syndrome Coronavirus (MERS-CoV) - were known to cause illness in humans.

In 2019, the coronavirus disease 2019 (COVID-19), caused by the Severe Acute Respiratory Syndrome coronavirus 2 (SARS-CoV-2), has emerged as a pandemic, claiming more than 3.9 million lives and around 180 million confirmed cases world-wide between December 2019 and June 2021. SARS-CoV-2 is a beta coronavirus that tends to cause severe illness in human beings, which is the reason for a huge, rapidly spreading outbreak of respiratory infections, including potentially fatal pneumonia.

Several different vaccines against COVID-19 were developed in a short-term and became available in early 2021. However, due to the rapid emergence of many variants of SARS-CoV-2 worldwide, there still is a need for improving the quality and the durability of the immune response induced in the vaccinated population. In particular, there remains a need for methods of generating robust immune responses, such as efficient cellular and/or humoral immune responses, and notably mucosal responses.

Furthermore, all vaccines against COVID-19 available today are delivered by injection, causing problems of safety, patient acceptability and morbidity, and making mass immunization more costly and less safe, particularly in resource-poor developing countries. Thus, there remains a genuine need for simple methods of vaccination, in particular by oral administration. Indeed, oral delivery offers a number of significant advantages over other routes of administration and notably compared with parenteral vaccines (e.g., simple administration and improved safety). However, even if the oral route represents the ideal means of delivering vaccines, the oral route is also the most difficult because of the numerous barriers posed by the gastrointestinal tract. In order to allow the effective immunization with protein vaccines, antigens must be protected, uptake enhanced and the immune response activated. Recently, the Inventors have shown that orally administered bioengineered virus-like particles (VLPs) displaying a protozoan variable surface protein (VSP) and model surface antigens, such as influenza virus hemagglutinin and neuraminidase, could generate immune responses that protect mice against influenza infection (Serradell et al., Nat. Commun. 10:361 (2019)). However, to date, no oral vaccines against COVID-19 are available.

The present invention is believed to meet the above needs by providing new viral-like particles (VLPs) displaying at their surface a protozoan variable surface protein (VSP) and a polypeptide antigen of a *Coronaviridae* virus.

Unpredictably, as each VSP/envelop protein combination has its own structural particularities that may affect their performance as immunogen, the Inventors have shown that such VLPs can be used to induce a robust immune response or to boost a previously-induced immune response against a *Coronaviridae* virus, via a mucosal route, such as oral or nasal administration. Furthermore, in a surprising manner, the Inventors have shown that this approach is able to induce a mucosal immunity, which would be particularly useful to control SARS-CoV-2 infection and dissemination.

In an aspect, the invention thus relates to a virus-like particle (VLP), in particular a retrovirus-derived virus-like particle, displaying at its surface at least:
- a variable surface protein (VSP), a VSP-like protein or a fragment thereof of a microorganism selected in the group consisting of *Giardia, Tetrahymena, Paramecium* and *Entamoeba* species, and
- a viral protein or a fragment thereof of a *Coronaviridae* virus.

As used herein, the term "Virus-Like Particle" (VLP) refers to a structure resembling a virus particle, which mimics the conformation of viruses. A virus-like particle in accordance with the invention is non-replicative since it lacks all or part of the viral genome, typically and preferably lacking all or part of the replicative and infectious components of the viral genome. The term "non-replicative", as used herein, refers to being incapable of replicating the genome comprised or not in the VLP. VLPs are multiprotein structures which are generally composed of one or more viral proteins. They can form (or "self-assemble") upon expression of a viral structural protein under appropriate circumstances. VLPs overcome some of the disadvantages of vaccines prepared using attenuated viruses because they can be produced without the need to have any live virus present during the production process. A wide variety of VLPs can be prepared. For example, VLPs including single or multiple capsid proteins either with or without envelope proteins and/or surface glycoproteins can be produced. In some cases, VLPs are non-enveloped and assemble by expression of just one major capsid protein. In other cases, VLPs are enveloped and can comprise multiple antigenic proteins found in the corresponding native virus. VLPs typically resemble their corresponding native virus and can display multivalent antigens.

Presentation of surface glycoproteins in the context of a VLP is advantageous for induction of neutralizing antibodies against an antigenic protein, such as a viral protein of a *Coronaviridae* virus, as compared to other forms of antigen presentation, e.g., soluble antigens not associated with a VLP. Neutralizing antibodies most often recognize tertiary or quaternary structures; this often requires presenting antigenic proteins, like envelope glycoproteins, in their native viral conformation. The proper presentation of an envelope glycoprotein at the surface of a VLP is hardly predictable. Some envelope glycoprotein may require their engineering for a better expression, such as the swap of their membrane and/or cytoplasmic tail with that of another viral protein, e.g., vesicular stomatitis virus G glycoprotein (VSV-G). However, this may affect the structure of the protein, notably its quaternary structure if in the wild type virus the envelope is expressed as a multimer. Furthermore, the co-expression of the VSP and the envelope glycoprotein onto the same membrane may result in interactions that modify the structure of the envelope glycoprotein, and therefore its capacity to trigger neutralizing antibodies (Nabs).

As used herein, the terms "variable surface protein", "VSP protein," or "VSP" refer to polypeptides that cover the entire surface of the *Giardia* parasite and are the major antigens recognized by the host immune system. VSP proteins are cysteine-rich proteins with multiple "CXXC" motifs (where X is any amino acid) that have several particular characteristics, including in some VSP the presence of CXC motifs, a *Giardia*-specific Zinc-finger motif, and GGCY motifs (Nash, Mol. Microbiol. 45:585-590 (2002); Adam et al., BMC Genomics 10:424 (2010)). More precisely, VSP proteins are type 1 integral membrane proteins that vary in size from 20 to 200 kDa; possess a variable amino-terminal cysteine-rich region (extracellular domain that represents the host/parasite interface and confers to the protein resistance to proteolytic digestion and low pH), and a conserved carboxy-terminal region that includes a hydrophobic transmembrane region and a short cytosolic tail comprising only 5 amino acids (CRGKA), which are not detected by the immune system. Only one VSP protein is expressed at any given time on the surface of each parasite (Nash, Philos. Trans. R. Soc. Lond. B. Biol. Sci. 352:1369-1375 (1997)). The *Giardia* VSPs and more particularly the extracellular domain of the *Giardia* VSPs comprise multiple CXXC motifs, preferably multiple CXXC motifs separated by several amino acids, from 3 to 20 amino acids and more particularly from 5 to 8 amino acids. Thus, in some embodiments, the VSP is a fragment, analog or derivative of a VSP or VSP-like protein from *Giardia,* wherein the VSP comprises 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 CXXC motifs. In some embodiments, the VSP comprises at least about 40, at least about 50, at least about 60, at least about 70, at least 80, about 90 or at least about 100 CXXC motifs from a *Giardia* VSP.

Within the context of the present invention, the terms "variable surface protein", "VSP protein," or "VSP proteins" include any VSP of the complete repertoire of *Giardia* VSP proteins, notably *Giardia lamblia.* Actually, *Giardia* parasites encodes a repertoire of about 200 genes encoding VSPs for the assemblage A (see, e.g., Morrison et al., Science 317:1921-1926 (2010); Adam et al., BMC Genomics 10:424 (2010)), and two reports of Svard's group describing the VSP repertoire of isolates derived from VSP assemblages B and E (Jerlstrom-Hultqvist et al., BMC Genomics 11:543 (2010); Franzen et al., PLoS Pathog. 5(8):cl000560 (2009)). The extracellular domain of a VSP allows the parasite to survive the hostile environment of the upper small intestine. VSPs are very resistant to variable pHs (reactivity to a conformational epitope by a monoclonal antibody directed to a particular VSP remains unaltered between pH 2 and 12), and digestion by trypsin and several other proteases. In addition, VSPs remain attached to the enteric mucosa (Rivero et al., Nat. Med. 16(5):551-7 (2010)).

It must be further noted that polypeptides comprising at least one CXXC motif, wherein C represents a cysteine residue and X any amino acid residue, such as *Giardia* VSPs or VSP-like proteins of other microorganisms may also be generated *in vitro* by genetic manipulation and produced in heterologous systems. Therefore, chemically- or cell-produced polypeptides, including those with amino acid variations not found in the wild type parasites (for instance variants of *Giardia* VSPs) are encompassed. VSPs may thus be prepared by any well-known procedure in the art, such as solid phase synthesis, liquid phase synthesis or genetic engineering.

VSPs used in the invention can undergo chemical modifications. Chemical modifications can be aimed at obtaining VSPs with increased protection against enzymatic degradation *in vivo,* and/or increased capacity to cross membrane barriers, thus increasing their half-lives and maintaining or improving their biological activity. Any chemical modification known in the art can be employed according to the present invention to modify a VSP. Such chemical modifications include but are not limited to:
(a) modifications to the N-terminal and/or C-terminal ends of the VSP proteins such as e.g., N-terminal acylation (preferably acetylation) or deamination, or modification of the C- terminal carboxyl group into an amide or an alcohol group; (b) modifications at the amide bond between two amino acids: acylation (preferably acetylation) or alkylation (preferably methylation) at the nitrogen atom or the alpha carbon of the amide bond linking two amino acids;
(c) modifications at the alpha carbon of the amide bond linking two amino acids such as, e.g., acylation (preferably acetylation) or alkylation (preferably methylation) at the alpha carbon of the amide bond linking two amino acids.
(d) chirality changes such as, e.g., replacement of one or more naturally occurring amino acids (L enantiomer) with the corresponding D-enantiomers;
(e) retro-inversions in which one or more naturally-occurring amino acids (L- enantiomer) are replaced with the corresponding D-enantiomers, together with an inversion of the amino acid chain (from the C-terminal end to the N-terminal end); and/or
(f) azapeptides wherein one or more alpha carbons are replaced with nitrogen atoms.

The terms "variable surface protein", "VSP protein," or "VSP" "as used herein also includes homologs (e.g., orthologs and paralogs) of "VSP" proteins from *Giardia,* VSP and VSP-like proteins found in other organisms, as well as fragments, variants, and derivatives thereof. The term "homolog," used with respect to a VSP protein or VSP-encoding gene of a first family or species, refers to distinct VSP protein or VSP-encoding genes of a second family or species which are determined by functional, structural, or genomic analyses to be a VSP protein or VSP-encoding gene of the second family or species which corresponds to the original VSP protein or VSP-encoding gene of the first family or species. As used herein, the term "homolog" refers to any VSP protein or VSP-encoding gene that is related to a reference VSP protein or VSP-encoding gene by descent from a common ancestral DNA sequence. The term homolog includes both orthologs and paralogs. The term "ortholog" refers to VSP homologs in different species that evolved from a common ancestral gene by speciation. Typically, orthologs retain the same or similar function despite differences in their primary structure (mutations). The term "paralog" refers to VSP homologs in the same species that evolved by genetic duplication of a common ancestral gene. In many cases, paralogs exhibit related (but not always identical functions). To the extent that a particular species has evolved multiple related genes from an ancestral DNA sequence shared with another species, the term ortholog can encompass the term paralog. Most often, homologs will have functional, structural, or genomic similarities. Techniques are known by which homologs of an enzyme or gene can readily be cloned using genetic probes and PC. Identity of cloned sequences as homologs can be confirmed using functional assays and/or by genomic mapping of the genes.

The "VSP-like proteins" or fragments thereof may be chosen among polypeptides derived from microorganisms other that *Giardia* sharing sequence homology and biochemical properties with *Giardia* and notably polypeptides containing multiple CXXC motifs, preferably multiple CXXC motifs separated by 5 to 8 amino acids. Indeed, alignments of the extracellular domain of *Giardia* VSP sequences (query) with other VSP-like molecules sequences have led to observe the presence of multiple CXXC motifs, notably separated by 5 to 8 amino acids, in proteins belonging to *Paramecium, Tetrahymena* and *Entamoeba* species. Thus, representative fragments of primary sequences of surface kinases of *Entamoeba* sp., and surface proteins of *Paramecium* sp. and *Tetrahymena* sp. predict a conserved domain containing CXXC motifs in a VSP-like architecture (compared with *Giardia* VSPs responsible for resistance to pH, temperature and proteolytic digestion).

In the present invention, the terms *"Giardia", "Tetrahymena","Paramecium"* and *"Entamoeba"* refers to microorganism genera, each of them comprising various species possibly infecting different hosts. For example, the genus *Giardia* comprises, but is not limited to, *Giardia lamblia* (also called *Giardia intestinalis* or *Giardia duodenalis), Giardia enterica, Giardia muris, Giardia simondi, Giardia bovis, Giardia canis, Giardia cati* and *Giardia microti.*

In an embodiment, the invention relates to a VLP as defined above, wherein the microorganism is selected from the group consisting of *Giardia lamblia, Tetrahymena thermophila, Paramecium tetraurelia* and *Entamoeba histolytica.*

In an embodiment, the microorganism is *Giardia lamblia.* In another embodiment, the microorganism is *Tetrahymena thermophila.* In another embodiment, the microorganism is *Entamoeba histolytica.* In another embodiment, the microorganism is *Paramecium tetraurelia.*

As used herein, the term "fragment of a protein" generally encompass any polypeptide comprising at least about 5, 6, 7, 8, 9, 10, 11, 12, 13, 14, 15, 16, 17, 18, 19, 20, 25, 30, 40, 50, 60, 70, 80, 90, 100, 150, 200, 300, 400, 500, 600, 700, 800, 900 or 1 000 contiguous or discontinuous amino acids of any designated protein or polypeptide, regardless it refers to a VSP or a viral protein. When the term "fragment of a protein" applies, in accordance with the present invention, to an antigen, such fragment should be capable of inducing the production of antibody, or stimulation of T cell, *in vivo.* Thus, a fragment of a protein should comprise at least one immunogenic epitope. When the term "fragment of a protein" applies, in accordance with the present invention, to the VSPs or VSP-like proteins of other microorganisms, such fragment should be capable of retaining the ability to attach to cells, particularly mucosal cells, more particularly epithelial cells of the gut and eventually inducing an immune response *per se.*

In a preferred embodiment, the VSP is the extracellular domain of a *Giardia* VSP or a fragment thereof (since said extracellular domain is the amino-terminal cysteine rich region comprising multiple CXXC motifs of the *Giardia* VSP protein). Indeed, the extracellular domain of a *Giardia* VSP is the domain resistant to the pH, temperature and proteolytic digestion. Accordingly, in another preferred embodiment, the polypeptide according to the invention comprises only the extracellular domain of a *Giardia* VSP or a fragment thereof. In such an embodiment, the transmembrane region and the cytoplasmic tail of a *Giardia* VSP are thus eliminated. It should be noted that the peptide signal may also be removed.

In an embodiment, the invention relates to a VLP as defined above, wherein the VSP, VSP-like protein or fragment thereof comprises or consists in an amino acid sequence selected from the group consisting of SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34, SEQ ID NO: 35, SEQ ID NO: 36, SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45, SEQ ID NO: 46, SEQ ID NO: 47, SEQ ID NO: 48, SEQ ID NO: 49, SEQ ID NO: 50, SEQ ID NO: 51, SEQ ID NO: 52, SEQ ID NO: 53, SEQ ID NO: 54, SEQ ID NO: 55, SEQ ID NO: 56, SEQ ID NO: 57, SEQ ID NO: 58, SEQ ID NO: 59, SEQ ID NO: 60, SEQ ID NO: 61, SEQ ID NO: 62, SEQ ID NO: 63, SEQ ID NO: 64, SEQ ID NO: 65, SEQ ID NO: 66, SEQ ID NO: 67, SEQ ID NO: 68, SEQ ID NO: 69, SEQ ID NO: 70, SEQ ID NO: 71, SEQ ID NO: 72, SEQ ID NO: 73, SEQ ID NO: 74, SEQ ID NO: 75, SEQ ID NO: 76, SEQ ID NO: 77, SEQ ID NO: 78, SEQ ID NO: 79, SEQ ID NO: 80, SEQ ID NO: 81, SEQ ID NO: 82, SEQ ID NO: 83, SEQ ID NO: 84, SEQ ID NO: 85, SEQ ID NO: 86, SEQ ID NO: 87, SEQ ID NO: 88, SEQ ID NO: 89, SEQ ID NO: 90, SEQ ID NO: 91, SEQ ID NO: 92, SEQ ID NO: 93, SEQ ID NO: 94, SEQ ID NO: 95, SEQ ID NO: 96, SEQ ID NO: 97, SEQ ID NO: 98, SEQ ID NO: 99, SEQ ID NO: 100, SEQ ID NO: 101, SEQ ID NO: 102, SEQ ID NO: 103, SEQ ID NO: 104, SEQ ID NO: 105, SEQ ID NO: 106, SEQ ID NO: 107, SEQ ID NO: 108, SEQ ID NO: 109, SEQ ID NO: 110, SEQ ID NO: 111, SEQ ID NO: 112, SEQ ID NO: 113, SEQ ID NO: 114, SEQ ID NO: 115, SEQ ID NO: 116, SEQ ID NO: 117, SEQ ID NO: 118, SEQ ID NO: 119, SEQ ID NO: 120, SEQ ID NO: 121, SEQ ID NO: 122, SEQ ID NO: 123 and SEQ ID NO: 124.

In a particular embodiment, the invention relates to a VLP as defined above, wherein the VSP, VSP-like protein or fragment thereof comprises or consists in an amino acid sequence selected from the group consisting of SEQ ID NO: 1, SEQ ID NO: 2, SEQ ID NO: 3, SEQ ID NO: 4 and SEQ ID NO: 5.

**Table 1. Non-limitative examples of VSP amino acid sequences from Giardia.**

| SEQ ID Numbers | Giardia Database Accession numbers | SEQ ID Numbers | Giardia Database Accession numbers |
|---|---|---|---|
| SEQ ID NO: 1 | GL50803_112208 (or VSP1267) | SEQ ID NO: 63 | GL50803_0050165 |
| SEQ ID NO: 2 | GL50803_113797 (or VSP417) | SEQ ID NO: 64 | GL50803_00112867 |
| SEQ ID NO: 3 | GL50803_00101074 (or VSP9B10) | SEQ ID NO: 65 | GL50803_0028626 |
| SEQ ID NO: 4 | GL50803_0040591 (orVSPA8) | SEQ ID NO: 66 | GL50803_0050169 |
| SEQ ID NO: 5 | GL50803_0050375 (or VSP7C9) | SEQ ID NO: 67 | GL50803_00103992 |
| SEQ ID NO: 6 | GL50803_0050370 | SEQ ID NO: 68 | GL50803_00137606 |
| SEQ ID NO: 7 | GL50803_00137614 | SEQ ID NO: 69 | GL50803_0050181 |
| SEQ ID NO: 8 | GL50803_00137612 | SEQ ID NO: 70 | GL50803_00137608 |
| SEQ ID NO: 9 | GL50803_00112113 | SEQ ID NO: 71 | GL50803_005812 |
| SEQ ID NO: 10 | GL50803_0013727 | SEQ ID NO: 72 | GL50803_00113357 |
| SEQ ID NO: 11 | GL50803_0041227 | SEQ ID NO: 73 | GL50803_00137620 |
| SEQ ID NO: 12 | GL50803_0050379 | SEQ ID NO: 74 | GL50803_00221693 |
| SEQ ID NO: 13 | GL50803_00115797 | SEQ ID NO: 75 | GL50803_00114162 |
| SEQ ID NO: 14 | GL50803_0050391 | SEQ ID NO: 76 | GL50803_00113024 |
| SEQ ID NO: 15 | GL50803_0050392 | SEQ ID NO: 77 | GL50803_0050225 |
| SEQ ID NO: 16 | GL50803_00112647 | SEQ ID NO: 78 | GL50803_0050229 |
| SEQ ID NO: 17 | GL50803_0089315 | SEQ ID NO: 79 | GL50803_0050259 |
| SEQ ID NO: 18 | GL50803_00113163 | SEQ ID NO: 80 | GL50803_00112801 |
| SEQ ID NO: 19 | GL50803_0087628 | SEQ ID NO: 81 | GL50803_00111933 |
| SEQ ID NO: 20 | GL50803_0038901 | SEQ ID NO: 82 | GL50803_00111936 |
| SEQ ID NO: 21 | GL50803_0050332 | SEQ ID NO: 83 | GL50803_00103142 |
| SEQ ID NO: 22 | GL50803_0011521 | SEQ ID NO: 84 | GL50803_00113304 |
| SEQ ID NO: 23 | GL50803_00117204 | SEQ ID NO: 85 | GL50803_0026894 |
| SEQ ID NO: 24 | GL50803_008595 | SEQ ID NO: 86 | GL50803_00116477 |
| SEQ ID NO: 25 | GL50803_00113439 | SEQ ID NO: 87 | GL50803_00114813 |
| SEQ ID NO: 26 | GL50803_00113450 | SEQ ID NO: 88 | GL50803_00137605 |
| SEQ ID NO: 27 | GL50803_0050345 | SEQ ID NO: 89 | GL50803_0014586 |
| SEQ ID NO: 28 | GL50803_00101010 | SEQ ID NO: 90 | GL50803_0050064 |
| SEQ ID NO: 29 | GL50803_00115047 | SEQ ID NO: 91 | GL50803_0050066 |
| SEQ ID NO: 30 | GL50803_0033279 | SEQ ID NO: 92 | GL50803_00112207 |
| SEQ ID NO: 31 | GL50803_00117472 | SEQ ID NO: 93 | GL50803_0050070 |
| SEQ ID NO: 32 | GL50803_00117473 | SEQ ID NO: 94 | GL50803_0013390 |
| SEQ ID NO: 33 | GL50803_00137729 | SEQ ID NO: 95 | GL50803_00137722 |
| SEQ ID NO: 34 | GL50803_0015400 | SEQ ID NO: 96 | GL50803_00137723 |
| SEQ ID NO: 35 | GL50803_0050359 | SEQ ID NO: 97 | GL50803_0037093 |
| SEQ ID NO: 36 | GL50803_00134710 | SEQ ID NO: 98 | GL50803_00137721 |
| SEQ ID NO: 37 | GL50803_0050363 | SEQ ID NO: 99 | GL50803_00101496 |
| SEQ ID NO: 38 | GL50803_0050366 | SEQ ID NO: 100 | GL50803_00137717 |
| SEQ ID NO: 39 | GL50803_00119706 | SEQ ID NO: 101 | GL50803_00137714 |
| SEQ ID NO: 40 | GL50803_00119707 | SEQ ID NO: 102 | GL50803_0011470 |
| SEQ ID NO: 41 | GL50803_00101380 | SEQ ID NO: 103 | GL50803_0016472 |
| SEQ ID NO: 42 | GL50803_0014831 | SEQ ID NO: 104 | GL50803_00112693 |
| SEQ ID NO: 43 | GL50803_0050289 | SEQ ID NO: 105 | GL50803_0041472 |
| SEQ ID NO: 44 | GL50803_0050290 | SEQ ID NO: 106 | GL50803_00137708 |
| SEQ ID NO: 45 | GL50803_00102178 | SEQ ID NO: 107 | GL50803_00137707 |
| SEQ ID NO: 46 | GL50803_00115830 | SEQ ID NO: 108 | GL50803_004313 |
| SEQ ID NO: 47 | GL50803_00115831 | SEQ ID NO: 109 | GL50803_00115742 |
| SEQ ID NO: 48 | GL50803_00137740 | SEQ ID NO: 110 | GL50803_0050100 |
| SEQ ID NO: 49 | GL50803_00137744 | SEQ ID NO: 111 | GL50803_0050101 |
| SEQ ID NO: 50 | GL50803_00137752 | SEQ ID NO: 112 | GL50803_0050102 |
| SEQ ID NO: 51 | GL50803_00136003 | SEQ ID NO: 113 | GL50803_00102662 |
| SEQ ID NO: 52 | GL50803_00136004 | SEQ ID NO: 114 | GL50803_0014331 |
| SEQ ID NO: 53 | GL50803_00101765 | SEQ ID NO: 115 | GL50803_00137617 |
| SEQ ID NO: 54 | GL50803_0050317 | SEQ ID NO: 116 | GL50803_0050116 |
| SEQ ID NO: 55 | GL50803_00114674 | SEQ ID NO: 117 | GL50803_00111732 |
| SEQ ID NO: 56 | GL50803_00137613 | SEQ ID NO: 118 | GL50803_00137681 |
| SEQ ID NO: 57 | GL50803_0050143 | SEQ ID NO: 119 | GL50803_0041401 |
| SEQ ID NO: 58 | GL50803_0016501 | SEQ ID NO: 120 | GL50803_00137604 |
| SEQ ID NO: 59 | GL50803_00137618 | SEQ ID NO: 121 | GL50803_00137697 |
| SEQ ID NO: 60 | GL50803_0014043 | SEQ ID NO: 122 | GL50803_0013194 |
| SEQ ID NO: 61 | GL50803_0032933 | SEQ ID NO: 123 | GL50803_0041626 |
| SEQ ID NO: 62 | GL50803_0050163 | SEQ ID NO: 124 | GL50803_0050390 |

In the present invention, the VSP, VSP-like protein or fragment thereof is exposed at the surface of the VLP. In this regard, the VSP, VSP-like protein or a fragment thereof may be exposed through binding to various structures, such as to an envelope protein or a fragment thereof, a synthetic linker, or through chemical or enzymatic reaction, including antibody.

In an embodiment, the VLP displays an envelope protein comprising at least a portion of the transmembrane domain of an envelope protein fused to the VSP, VSP-like protein or a fragment thereof.

In an embodiment, the VLP displays an envelope protein of a virus, such as an envelope protein from a vesicular stomatitis virus (VSV) or a fragment thereof (e.g. the transmembrane (TM) region and/or the cytoplasmic tail (CT) of VSV G glycoprotein), fused with the VSP, VSP-like protein or a fragment thereof exposed at the surface of the VLP, for instance by genetic or chemical fusion with the VSV envelope protein or a fragment thereof.

In an embodiment, the invention relates to a VLP as defined above, wherein the VSP, VSP-like protein or fragment thereof, preferably the extracellular region of the VSP, is fused with the transmembrane region of the VSV-G glycoprotein.

In an embodiment, the VSP, VSP-like protein or fragment thereof comprises or consists in SEQ ID NO: 125.

**Table 2. Example of VSP fused to VSV-G.**

| | | |
|---|---|---|
| SEQ ID NO: 125 | VSP-VSV-G | MLLIAFYLILSTFAVDCKNSGNSCEAGQCDTIGDTEICMQCNQGKVPINGICTAH SEEAVTNAGCKKNGGTNIEESDKVCGQCGNGYFLHKGGCYKIGEAPGNLICADE ASNPGARTAGVCGACKDGYYKNSDAVATADSCIACEDANCATCGGAGENKCTK CIDGYFVGATGNEGGCIKCDATTGPNSYKGVAGCAKCEKPKNAGPAKCIECAAD YLKTEADEQTSCVSEAVCREGKTHFPTTDSAGGNKKVCVSCGTTNNGGIENCGE CTSKESAARAGTEITCTKCSSNNLSPLGDACLTDCPAGTYAVSGDSGSVCKPCHN TCAGCQTDDRETSCTACYPGYSLLYESNGATGRCVKECTGAFITNCADGQCTAN VGGAKYCTQCKDGYAPIDGICTAVAAAGRDVSVCTATGGKCTACTGNYALLSG GCYNTQTLPGKSVCKAVANSNDGKCKTCANGQAPDPATNFCPLCDSTCAECST KNDADACTKCFPGYYKTGNKCIKCTESSNNGKKIDGIPDCLSCEAPINTGPVICYV |
| | | KTDGTSDDNSGNGGDSTNKSGLSTGGSEQKLISEEDLSGGGGSKSSIASFFFIIGLI IGLFLVLRVGIHLCIKLKHTKKRQIYTDIEMNRLGK |

In an embodiment, the invention relates to a VLP as defined above, wherein the viral protein or the fragment thereof of a *Coronaviridae* virus, is fused with the transmembrane region of the VSV-G glycoprotein.

In another particular embodiment, the envelope may be functionalized, thereby allowing the binding, to the envelope, through covalent or non-covalent interaction, of any selected molecule of interest. The functionalized envelope may comprise a linker wherein the linker allows (specific) binding of any selected molecule of interest. As an example, the envelope may comprise an avidine or biotine moiety, allowing specific binding thereto of a molecule.

The expression of capsid protein Gag in eukaryotic cells suffices to generate genome free retroviral particles. In an embodiment, the VLP comprises a retroviral or lentiviral Gag protein (such as the murine leukemia virus (MLV) or HIV Gag proteins, or fragments thereof), even more preferably a modified retroviral Gag protein.

In the present invention, the VLP displays (i.e., comprises on the outside face of the VLP) a viral protein or a fragment thereof of a *Coronaviridae* virus.

Indeed, the VLP of the invention favors a correct delivery of an antigenic protein from a *Coronaviridae* virus into the mucosa without suffering extensive degradation in the digestive track. This viral protein (or fragment thereof) is used as an antigen in order to induce an immune response against *Coronaviridae* viruses. Viruses of the *coronaviridae* family include *letovirinae* and *orthocoronavirinae. Letovirinae* include alpaletovirus. *Orthocoronavirinae* include alpha coronavirus, beta coronavirus, delta coronavirus and gamma coronavirus. For example, the coronavirus may be selected from the group consisting of Severe Acute Respiratory Syndrome (SARS) coronaviruses, including SARS-CoV and SARS-CoV-2 (also called COVID-19), Middle East Respiratory syndrome-related (MERS) coronavirus, human coronavirus OC43 (HCoV-OC43), human coronavirus 229E (HCoV-229E), Human Coronavirus NL63 (HCoV-NL63), Human Coronavirus HKU1 (HCoV-HKU1) and variants thereof.

In an embodiment, the *Coronaviridae* virus is a coronavirus, preferably selected from the group consisting of SARS-CoV, SARS-CoV-2, MERS-CoV, HCoV-OC43, HCoV-229E, HCoV-NL63, HCoV-HKU1 and variants thereof.

In a most preferred embodiment, the coronavirus is SARS-CoV-2 or a variant thereof.

SARS-CoV-2 has many variants. Most changes have little to no impact on the virus' properties. However, some changes may affect the virus's properties, such as how easily it spreads, the associated disease severity, or the performance of therapeutic medicines, diagnostic tools, or other public health and social measures.

In an embodiment, variants of SARS-CoV-2 are selected from the group comprising Alpha, Beta, Gamma, Delta, Epsilon, Zeta, Eta, Theta, Iota and Kappa variants, as labelled by the World health Organization (WHO).

In an embodiment, the invention relates to a VLP as defined above, wherein the viral protein is a structural protein of SARS-CoV-2. Structural proteins of SARS-CoV-2 are the Spike (S) protein, the membrane (M) protein, the envelope (E) protein and the nucleocapsid (N) protein.

The SARS-CoV-2 Spike glycoprotein (or S protein) mediates the virus attachment to host cell surface receptors ACE2 and facilitates virus entry by assisting fusion between viral and host cell membranes. It is the most exposed and immunogenic viral protein. The Spike glycoprotein of SARS-CoV-2 is a trimeric macromolecule with two furin-like protease cleavage sites. One of the sites is at the boundary between S1 and S2 subunits having poly-basic residues, which is characteristic of SARS-CoV-2. The other cleavage site is located within the S2 subunit.

The SARS-CoV-2 membrane glycoprotein (or M protein) is conserved across the β-coronaviruses. It has a triple transmembrane domain and promotes spike installation.

The SARS-CoV-2 Envelop protein (or E-protein) is conserved across β-coronaviruses. It is a tiny integral membrane protein that pentamerizes to form ionic pore across the membrane called viroporins. It is essential for viral assembly and release.

The SARS-CoV-2 nucleocapsid protein (or N protein) is expressed in the early stage of infection and is the most abundant protein. It forms a core of a ribonucleoprotein by binding to viral RNA. It helps RNA to enter in the cell and to interact with cellular components.

In an embodiment, the invention relates to a VLP as defined above, wherein the viral protein is selected from the group consisting of S protein, M protein, E protein and N protein, preferably S protein.

In non-limitative examples, the invention relates to a VLP as defined above, wherein:
- S protein comprises or consists in an amino acid sequence selected from the group consisting of SEQ ID NO: 126, SEQ ID NO: 127, SEQ ID NO: 128, SEQ ID NO: 129, SEQ ID NO: 130 and SEQ ID NO: 131,
- M protein comprises or consists in an amino acid sequence selected from the group consisting of SEQ ID NO: 132,
   and- N protein comprises or consists in an amino acid sequence selected from the group consisting of SEQ ID NO: 133.

**Table 3. Non-limitative examples of amino acid sequences of viral proteins of, or derived from, SARS-CoV-2, which can be used as antigens in the present invention. S: Spike protein; Variant D614G: also known as Wuhan variant; Sst1: artificial variant 1 of Spike; modCT: modification in the cytoplasmic tail; N: nucleocapsid protein; M: membrane glycoprotein.**

| | | |
|---|---|---|
| SEQ ID NO: 126 | SCoV2_ S protein Wild-Type | MVVFLVLLPLVSSQCVNLTTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLGVYYHKNNKSWMESEFRVYSSANNCTFEYVSQPFLMDLEGKQGNFKNLREF VFKNIDGYFKIYSKHTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEKGIYQTSNFRVQPTE SIVRFPNITNLCPFGEVFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNNLDSKVGGNYNYLY RLFRKSNLKPFERDISTEIYQAGSTPCNGVEGFNCYFPLQSYGFQPTNGVGYQPYRVVVLSFELL HAPATVCGPKKSTNLVKNKCVNFNFNGLTGTGVLTESNKKFLPFQQFGRDIADTTDAVRDPQT LEILDITPCSFGGVSVITPGTNTSNQVAVLYQDVNCTEVPVAIHADQLTPTWRVYSTGSNVFQT RAGCLIGAEHVNNSYECDIPIGAGICASYQTQTNSPRRARSVASQSIIAYTMSLGAENSVAYSNN SIAIPTNFTISVTTEILPVSMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFN GIGVTQNVLYENQKLIANQFNSAIGKIQDSLSSTASALGKLQDVVNQNAQALNTLVKQLSSNFG AISSVLNDILSRLDKVEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPD VDLGDISGINASVVNIQKEIDRLNEVAKNLNESLIDLQELGKYEQGSYIKWPWYIWLGFIAGLIAI VMVTIMLCCMTSCCSCLKGCCSCGSCCKFDEDDSEPVLKGVKLHYT |
| SEQ ID NO: 127 | SCoV2_ S protein Variant D614G | MVVFLVLLPLVSSQCVNLTTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLGVYYHKNNKSWMESEFRVYSSANNCTFEYVSQPFLMDLEGKQGNFKNLREF VFKNIDGYFKIYSKHTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEKGIYQTSNFRVQPTE SIVRFPNITNLCPFGEVFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNNLDSKVGGNYNYLY RLFRKSNLKPFERDISTEIYQAGSTPCNGVEGFNCYFPLQSYGFQPTNGVGYQPYRVVVLSFELL HAPATVCGPKKSTNLVKNKCVNFNFNGLTGTGVLTESNKKFLPFQQFGRDIADTTDAVRDPQT LEILDITPCSFGGVSVITPGTNTSNQVAVLYQGVNCTEVPVAIHADQLTPTWRVYSTGSNVFQT RAGCLIGAEHVNNSYECDIPIGAGICASYQTQTNSPRRARSVASQSIIAYTMSLGAENSVAYSNN SIAIPTNFTISVTTEILPVSMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFN |
| | | GIGVTQNVLYENQKLIANQFNSAIGKIQDSLSSTASALGKLQDVVNQNAQALNTLVKQLSSNFG AISSVLNDILSRLDKVEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPD VDLGDISGINASVVNIQKEIDRLNEVAKNLNESLIDLQELGKYEQGSYIKWPWYIWLGFIAGLIAI VMVTIMLCCMTSCCSCLKGCCSCGSCCKFDEDDSEPVLKGVKLHYT |
| SEQ ID NO: 128 | SCoV2_ S protein Wild-Type modCT | MVVFLVLLPLVSSQCVNLTTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLGVYYHKNNKSWMESEFRVYSSANNCTFEYVSQPFLMDLEGKQGNFKNLREF VFKNIDGYFKIYSKHTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEKGIYQTSNFRVQPTE SIVRFPNITNLCPFGEVFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNNLDSKVGGNYNYLY RLFRKSNLKPFERDISTEIYQAGSTPCNGVEGFNCYFPLQSYGFQPTNGVGYQPYRVVVLSFELL HAPATVCGPKKSTNLVKNKCVNFNFNGLTGTGVLTESNKKFLPFQQFGRDIADTTDAVRDPQT LEILDITPCSFGGVSVITPGTNTSNQVAVLYQDVNCTEVPVAIHADQLTPTWRVYSTGSNVFQT RAGCLIGAEHVNNSYECDIPIGAGICASYQTQTNSPRRARSVASQSIIAYTMSLGAENSVAYSNN SIAIPTNFTISVTTEILPVSMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFN GIGVTQNVLYENQKLIANQFNSAIGKIQDSLSSTASALGKLQDVVNQNAQALNTLVKQLSSNFG AISSVLNDILSRLDKVEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPD VDLGDISGINASVVNIQKEIDRLNEVAKNLNESLIDLQELGKYEQGSYIKWPWYIWLGFIAGLIAI VMVTIMLCCMTSCCSCLKGCCSCGSCCKFDEDDSEPVLKGVALAYT |
| SEQ ID NO: 129 | SCoV2_ S protein Variant D614G modCT | MVVFLVLLPLVSSQCVNLTTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLGVYYHKNNKSWMESEFRVYSSANNCTFEYVSQPFLMDLEGKQGNFKNLREF VFKNIDGYFKIYSKHTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEKGIYQTSNFRVQPTE SIVRFPNITNLCPFGEVFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNNLDSKVGGNYNYLY RLFRKSNLKPFERDISTEIYQAGSTPCNGVEGFNCYFPLQSYGFQPTNGVGYQPYRVVVLSFELL HAPATVCGPKKSTNLVKNKCVNFNFNGLTGTGVLTESNKKFLPFQQFGRDIADTTDAVRDPQT LEILDITPCSFGGVSVITPGTNTSNQVAVLYQGVNCTEVPVAIHADQLTPTWRVYSTGSNVFQT RAGCLIGAEHVNNSYECDIPIGAGICASYQTQTNSPRRARSVASQSIIAYTMSLGAENSVAYSNN SIAIPTNFTISVTTEILPVSMTKTSVDCTMYICGDSTECSNLLLQYGSFCTQLNRALTGIAVEQDKN |
| | | TQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFN GIGVTQNVLYENQKLIANQFNSAIGKIQDSLSSTASALGKLQDVVNQNAQALNTLVKQLSSNFG AISSVLNDILSRLDKVEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPD VDLGDISGINASVVNIQKEIDRLNEVAKNLNESLIDLQELGKYEQGSYIKWPWYIWLGFIAGLIAI VMVTIMLCCMTSCCSCLKGCCSCGSCCKFDEDDSEPVLKGVALAYT |
| SEQ ID NO: 130 | SCoV2 Sst1 modCT | MVVFLVLLPLVSSQCVNLTTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLGVYYHKNNKSWMESEFRVYSSANNCTFEYVSQPFLMDLEGKQGNFKNLREF VFKNIDGYFKIYSKHTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEKGIYQTSNFRVQPTE SIVRFPNITNLCPFGEVFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNNLDSKVGGNYNYLY RLFRKSNLKPFERDISTEIYQAGSTPCNGVEGFNCYFPLQSYGFQPTNGVGYQPYRVVVLSFELL HAPATVCGPKKSTNLVKNKCVNFNFNGLTGTGVLTESNKKFLPFQQFGRDIADTTDAVRDPQT LEILDITPCSFGGVSVITPGTNTSNQVAVLYQDVNCTEVPVAIHADQLTPTWRVYSTGSNVFQT RAGCLIGAEHVNNSYECDIPIGAGICASYQTQTNSPGSASSVASQSIIAYTMSLGAENSVAYSNN SIAIPTNFTISVTTEILPVSMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFN GIGVTQNVLYENQKLIANQFNSAIGKIQDSLSSTASALGKLQDVVNQNAQALNTLVKQLSSNFG AISSVLNDILSRLDPPEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPD VDLGDISGINASVVNIQKEIDRLNEVAKNLNESLIDLQELGKYEQEGSYIKWPWYIWLGFIAGLIA IVMVTIMLCCMTSCCSCLKGCCSCGSCCKFDEDDSEPVLKGVALAYT |
| SEQ ID NO: 131 | SCoV2 Sst1 D614G modCT | MVVFLVLLPLVSSQCVNLTTRTQLPPAYTNSFTRGVYYPDKVFRSSVLHSTQDLFLPFFSNVTWF HAIHVSGTNGTKRFDNPVLPFNDGVYFASTEKSNIIRGWIFGTTLDSKTQSLLIVNNATNVVIKV CEFQFCNDPFLGVYYHKNNKSWMESEFRVYSSANNCTFEYVSQPFLMDLEGKQGNFKNLREF VFKNIDGYFKIYSKHTPINLVRDLPQGFSALEPLVDLPIGINITRFQTLLALHRSYLTPGDSSSGWT AGAAAYYVGYLQPRTFLLKYNENGTITDAVDCALDPLSETKCTLKSFTVEKGIYQTSNFRVQPTE SIVRFPNITNLCPFGEVFNATRFASVYAWNRKRISNCVADYSVLYNSASFSTFKCYGVSPTKLNDL CFTNVYADSFVIRGDEVRQIAPGQTGKIADYNYKLPDDFTGCVIAWNSNNLDSKVGGNYNYLY RLFRKSNLKPFERDISTEIYQAGSTPCNGVEGFNCYFPLQSYGFQPTNGVGYQPYRVVVLSFELL HAPATVCGPKKSTNLVKNKCVNFNFNGLTGTGVLTESNKKFLPFQQFGRDIADTTDAVRDPQT LEILDITPCSFGGVSVITPGTNTSNQVAVLYQGVNCTEVPVAIHADQLTPTWRVYSTGSNVFQT |
| | | RAGCLIGAEHVNNSYECDIPIGAGICASYQTQTNSPGSASSVASQSIIAYTMSLGAENSVAYSNN SIAIPTNFTISVTTEILPVSMTKTSVDCTMYICGDSTECSNLLLQYGSFCTQLNRALTGIAVEQDKN TQEVFAQVKQIYKTPPIKDFGGFNFSQILPDPSKPSKRSFIEDLLFNKVTLADAGFIKQYGDCLGDI AARDLICAQKFNGLTVLPPLLTDEMIAQYTSALLAGTITSGWTFGAGAALQIPFAMQMAYRFN GIGVTQNVLYENQKLIANQFNSAIGKIQDSLSSTASALGKLQDVVNQNAQALNTLVKQLSSNFG AISSVLNDILSRLDPPEAEVQIDRLITGRLQSLQTYVTQQLIRAAEIRASANLAATKMSECVLGQS KRVDFCGKGYHLMSFPQSAPHGVVFLHVTYVPAQEKNFTTAPAICHDGKAHFPREGVFVSNG THWFVTQRNFYEPQIITTDNTFVSGNCDVVIGIVNNTVYDPLQPELDSFKEELDKYFKNHTSPD VDLGDISGINASVVNIQKEIDRLNEVAKNLNESLIDLQELGKYEQEGSYIKWPWYIWLGFIAGLIA IVMVTIMLCCMTSCCSCLKGCCSCGSCCKFDEDDSEPVLKGVALAYT |
| SEQ ID NO: 132 | SCoV2_ N protein Wild-Type | MSDNGPQNQRNAPRITFGGPSDSTGSNQNGERSGARSKQRRPQGLPNNTASWFTALTQHG KEDLKFPRGQGVPINTNSSPDDQIGYYRRATRRIRGGDGKMKDLSPRWYFYYLGTGPEAGLPY GANKDGIIWVATEGALNTPKDHIGTRNPANNAAIVLQLPQGTTLPKGFYAEGSRGGSQASSRS SSRSRNSSRNSTPGSSRGTSPARMAGNGGDAALALLLLDRLNQLESKMSGKGQQQQGQTVTK KSAAEASKKPRQKRTATKAYNVTQAFGRRGPEQTQGNFGDQELIRQGTDYKHWPQIAQFAPS ASAFFGMSRIGMEVTPSGTWLTYTGAIKLDDKDPNFKDQVILLNKHIDAYKTFPPTEPKKDKKK KADETQALPQRQKKQQTVTLLPAADLDDFSKQLQQSMSSADSTQA |
| SEQ ID NO: 133 | SCoV2_ M protein Wild-Type | MADSNGTITVEELKKLLEQWNLVIGFLFLTWICLLQFAYANRNRFLYIIKLIFLWLLWPVTLACFV LAAVYRINWITGGIAIAMACLVGLMWLSYFIASFRLFARTRSMWSFNPETNILLNVPLHGTILTR PLLESELVIGAVILRGHLRIAGHHLGRCDIKDLPKEITVATSRTLSYYKLGASQRVAGDSGFAAYSR YRIGNYKLNTDHSSSSDNIALLVQ |

In an embodiment, the VLP of the invention displays at its surface:
- a VSP, a VSP-like protein or a fragment thereof of *Giardia lamblia,* and
- a S protein or a fragment thereof of SARS-CoV-2.

In an embodiment, the invention relates to a VLP as defined above, wherein the viral protein of a *Coronaviridae* virus is glycosylated.

In an embodiment, the invention relates to a VLP as defined above, wherein the viral protein of a *Coronaviridae* virus is the S protein of SARS-CoV-2, said S protein being glycosylated.

Modulation of glycosylation is often exploited by pathogens for immune evasion. Masking epitopes on protein immunogens with "self" glycans can also shield the underlying protein surface from humoral immune surveillance. Applying glycosylation to the viral antigen is an efficient strategy to improve elicitation of broadly neutralizing antibodies that incorporate glycans into their binding epitopes.

In an embodiment, the invention relates to a VLP as defined above, wherein the viral protein of a *Coronaviridae* virus protein is stabilized in pre-fusion conformation.

In an embodiment, the invention relates to a VLP as defined above, wherein the viral protein of a *Coronaviridae* virus is the S protein of SARS-CoV-2, said S protein being stabilized in pre-fusion conformation.

A viral protein in pre-fusion conformation is in the conformational state it is in before the envelope of the virus fuses with the membrane of the cell it infects. Prefusion-stabilized viral proteins are often superior immunogens as compared to their wild-type counterparts. Moreover, prefusion stabilization tends to increase the recombinant expression of viral fusion glycoproteins, possibly by preventing misfolding that results from a tendency to adopt the more stable postfusion structure.

In an embodiment, the VLP of the invention displays at its surface at least two distinct viral proteins, preferably S and M proteins.

In an embodiment, the VLP of the invention displays at its surface at least three distinct viral proteins, preferably S, M and N proteins.

In an embodiment, the VLP of the invention further comprises a Gag protein fused to its carboxy terminus to an additional antigen from a *Coronaviridae* virus. In an embodiment, the VLP of the invention further displays a N protein or a fragment thereof fused to a Gag protein. In a particular embodiment, the first (aa 5-102) and second half (aa 242-353) of N is fused to the Gag protein. In an embodiment, the VLP of the invention further comprises a Gag protein fused to its carboxy terminus to the N protein or a fragment thereof from a *Coronaviridae* virus, preferably from SARS-CoV-2. This provides an additional excellent T-cell response against the fused N protein or fragment thereof.

In a preferred embodiment, the VLP of the invention displays at its surface:
- a VSP, a VSP-like protein or a fragment thereof of *Giardia lamblia,*
- a S protein or a fragment thereof of SARS-CoV-2, preferably stabilized in pre-fusion conformation, and
- possibly a N protein or a fragment thereof of SARS-CoV-2 fused to a Gag protein.

In a preferred embodiment, the VLP of the invention displays at its surface:
- a VSP, a VSP-like protein or a fragment thereof of *Giardia lamblia,*
- a S protein or a fragment thereof of SARS-CoV-2, preferably stabilized in pre-fusion conformation,
- a M protein or a fragment thereof of SARS-CoV-2, and
- possibly a N protein or a fragment thereof of SARS-CoV-2 fused to a Gag protein.

All the embodiments and features given above for the VLPs of the invention apply *mutatis mutandis* to the other aspects of the invention described below which involve said VLPs.

In another aspect, the invention relates to a pharmaceutical composition comprising a VLP as defined above as active ingredient.

In an embodiment, the pharmaceutical composition of the invention is a vaccine.

In an embodiment, the pharmaceutical composition of the invention further comprises at least one pharmaceutically acceptable excipient.

"Pharmaceutically acceptable" refers to molecular entities and compositions that do not produce an adverse, allergic or other untoward reaction when administered to a mammal, especially a human, as appropriate. A pharmaceutically acceptable excipient refers to a non-toxic solid, semi-solid or liquid filler, diluent, encapsulating material or formulation auxiliary of any type.

Such pharmaceutically acceptable excipients include, but are not limited to, water, salts, buffers, adjuvants, carriers or other substances which are desirable for improving the efficacy of the composition. Examples of pharmaceutically materials suitable for use in preparation of pharmaceutical compositions are provided in numerous sources including REMINGTON'S PHARMACEUTICAL SCIENCES (Osol, A, ed., Mack Publishing Co., (1990)).

Furthermore, the pharmaceutical composition may comprise additional adjuvants or immunogenic or biologically active agents. For example, the pharmaceutical composition can further comprise adjuvant(s) selected from aluminium, in particular aluminium phosphate, AS03, MF59 and AS04.

However, in other embodiments, the pharmaceutical composition is devoid of adjuvant, since an advantageous feature of the invention is the high immunogenicity of the composition, even in the absence of adjuvants. The absence of an adjuvant, furthermore, minimizes the occurrence of unwanted inflammatory T-cell responses representing a safety concern in the vaccination.

The pharmaceutical composition of the invention is preferably formulated for an oral or a mucosal administration. The doses used for the oral or a mucosal administration can be adapted as a function of various parameters, and in particular as a function of the mode of the relevant pathology, or alternatively of the desired duration of treatment. The pharmaceutical composition may be administered to any suitable mucosa, and the administration includes oral (via the mucosa of the digestive system), nasal, vaginal, sublingual, ocular, rectal, urinal, intramammal, pulmonal, otolar (i.e., via the ear) and buccal administration, preferably buccal or sublingual administration (oromucosal administration). In a preferred embodiment, the pharmaceutical composition is formulated for oral or nasal administration.

Upon formulation, pharmaceutical compositions will be administered in a manner compatible with the dosage formulation and in such amount as is therapeutically effective. The formulations are easily administered in a variety of dosage forms, such as the type of, e.g., tablets or other solids for oral or a mucosal administration; time release capsules; and any other form currently used. Accordingly, the pharmaceutical composition may be in the form of a spray, an aerosol, a mixture, a suspension, a dispersion, an emulsion, a gel, a paste, a syrup, a cream, an ointment, implants (ear, eye, skin, nose, rectal, and vaginal), intramammary preparations, vagitories, suppositories, or uteritories). In certain embodiments, the use of liposomes is contemplated. The formation and use of liposomes are known to those of skill in the art.

More particularly, the pharmaceutical composition is formulated so that the VLP, and in in particular the viral protein displayed at its surface, is resistant to enzymatic and chemical degradation of the upper gastrointestinal tract, when necessary.

In another aspect, the invention relates to a VLP as defined above for use as a medicament.

The present invention also relates to a method of prevention and/or treatment of a viral infection in a subject in need thereof, comprising administering to said subject an effective amount of a VLP as defined above.

The present invention also relates to the use of a VLP as defined above in the manufacture of a medicament.

As used herein, the term "subject" denotes a mammal, such as a rodent, a feline, a canine, and a primate. Preferably, a subject according to the invention is a human.

In the context of the invention, the term "treating" or "treatment", as used herein, means reversing, alleviating, inhibiting the progress of, or preventing the disease or the infection to which such term applies, or one or more symptoms of such disease or infection.

Within the meaning of the invention, the term "preventing" or "prevention" with respect to a disease or an infection is intended to mean the decrease of a risk of occurrence of said disease or infection.

An "effective amount" is intended for a minimal amount of VLP which is necessary to impart therapeutic or prophylactic benefit to a subject. For example, an effective amount to a mammal is such an amount which induces, ameliorates or otherwise causes an improvement in the infection symptoms, disease progression or physiological conditions associated with or resistance to succumbing to an infection.

In another aspect, the invention relates to a VLP as defined above for use in the treatment or prevention of an infection by a virus from the *Coronaviridae* family.

The present invention also relates to a method of prevention and/or treatment of an infection by a virus from the *Coronaviridae* family in a subject in need thereof, comprising administering to said subject an effective amount of a VLP as defined above.

The present invention also relates to the use of a VLP as defined above in the manufacture of a medicament to prevent and/or treat an infection by a virus from the *Coronaviridae* family.

In another aspect, the invention relates to a VLP as defined above for use as a vaccine against an infection by a virus from the *Coronaviridae* family.

The present invention also relates to a method of vaccination (or immunization) of a subject against a virus from the *Coronaviridae* family, comprising administering to said subject an effective amount of a VLP as defined above.

The present invention also relates to the use of a VLP as defined above in the manufacture of a vaccine against a virus from the *Coronaviridae* family.

In one preferred embodiment, the pharmaceutical composition is administrated by mucosal route.

In an embodiment, the VLP of the invention is orally or intranasally administered to a patient.

In an embodiment, the VLP of the invention is administered as a boost to a patient who has previously received a vaccine, preferably an adenovirus-based vaccine, by subcutaneous or intramuscular injection, against the virus from the *Coronaviridae* family.

In an embodiment, the VLP of the invention is administered twice to the patient.

In an embodiment, the VLP of the invention is administered thrice or more to the patient.

In an embodiment, the patient receives a first administration of the VLP of the invention and a second administration of the VLP of the invention, both administrations being spaced at least one week apart, preferably at least two weeks apart, more preferably at least three to four weeks apart.

In an embodiment, the VLP is administered to a patient to induce the production of immunoglobulins A (IgA) by the mucosal surfaces of the aerodigestive tract, in particular in the bronchi and pulmonary alveoli. The production of IgA can be measured by detecting IgA in bronchoalveolar lavage liquid, as shown in the examples.

The following figures and examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

**Figure 1****.** Immunogenicity of different variants of Spike in mice. Swt: S wild-type of SARS-CoV-2 (original Wuhan strain Acc. No. NC_045512 and its variant D614G). Sst1: S artificial subtype 1 (K986P/V987P, 682RRAR-685GSAS (modFurinCS)). Sst2: S artificial subtype 2 (T791C/A879C, 682RRAR-685GSAS (modFurinCS)). Sst3: S artificial subtype 3 (S884C/A893C, 682RRAR-685GSAS (modFurinCS)). Sst4: S artificial subtype 4 (G885C/Q913C, 682RRAR-685GSAS (modFurinCS)). Sst5: S artificial subtype 5 (S884C/Q913C, 682RRAR-685GSAS (modFurinCS)).
**Figure 2****.** Serum antibody responses to oral administration of different vaccine formulations in hamsters.
**Figure 3****.** Serum antibody responses to intramuscular administration of different vaccine formulations in hamsters.
**Figure 4****.** Bronchoalveolar lavage IgA responses.
**Figure 5****.** Neutralizing antibodies against SARS-CoV-2 entry.
**Figure 6****.** Bronchoalveolar IgA after oral boost of intramuscularly vaccinated hamsters.
**Figure 7****.** Vaccinated hamsters challenged with SARS-CoV-2.

### EXAMPLES

The following experiments were conducted to design and evaluate a thermostable orally administered enveloped virus-like-particle (e-VLP) vaccine against SARS-CoV-2.

e-VLPs are particularly appropriate to induce a robust neutralizing immune response. Indeed, e-VLPs have the same lipid membrane than the cell they derive from. Likewise, virus envelope proteins that VLPs express have the same conformation that they have on the lipid membrane of an infected cell, and that the virus itself. As neutralizing antibodies (Nabs) are mostly targeted to conformational structures, e-VLPs are particularly suitable for Nab induction. Moreover, e-VLPs can be harnessed with variable surface proteins (VSP) from the intestinal parasite *Giardia lamblia,* affording them resistance after oral administration.

VSP-decorated e-VLPs (VSP-e-VLPs) expressing various forms of the S protein, with or without M expression, were produced and tested. Unexpectedly, it was found that VSP-e-VLPs expressing a pre-fusion stabilized form of S, and M, trigger robust mucosal Nabs against SARS-CoV-2 in mice and hamsters, which translate into complete protection from a viral challenge. Such a vaccine could be part of the arsenal against SARS-CoV-2, in a prime-boost vaccine strategy or as a boost for existing vaccine based on platforms (such as adenoviral ones) that do not allow efficient homologous boost and mRNA-based vaccines that do not afford infections, although they are highly efficient against severe forms of the disease.

### Materials & Methods

**Viruses.** SARS-CoV-2 isolates were propagated in VeroE6 cells in Opti-MEM I (Invitrogen) containing 0.3% bovine serum albumin (BSA) and 1 µg of L-1-tosylamide-2-phenylethyl chloromethyl ketone treated-trypsin per mL at 37 °C. All experiments with SARS-CoV-2 were performed in a biosafety level 3 (BSL3) containment laboratory at the CIDIE CONICET (Argentina) or in enhanced BSL3 containment laboratories at the Sorbonne University (France).

**Experimental animals.** For immunization and challenge experiments, the group sizes were chosen based on previous experience and littermates of the same sex were randomly assigned. The number of animals for each experiment and all procedures followed the protocols approved by the Institutional Committee for Care and Use of Experimental Animals. One-month-old, five females and five males, SPF Golden Syrian hamsters per group were used in the challenge experiments and 6 month-old, 5 females and 5 males, Golden Syrian hamsters per group were used in the immunization studies. For challenge experiments, under ketamine-xylazine anesthesia, ten hamsters per group were inoculated with 10⁵ PFU of SARS-CoV-2 (in 100 µL) or PBS (mock) via the intranasal route. Baseline body weights were measured before infection and body weight was monitored for 28 days. No animals were harmed during the collection of blood.

**Neutralization assays**. Serum taken from intranasally inoculated hamsters at 14 dpi was tested for viral neutralizing antibody titer by microneutralizing assay in Vero E6 cells. Briefly, the 50-fold serial diluted (1:50 to 1:5000) serum samples were mixed with 100 TCID50 of SARS-CoV-2 virus and incubated at 37°C for 1 hour. The mixture was then added to Vero E6 cells and further incubated at 37°C for 72 hours. The neutralizing antibody titer was defined as the highest dilution that inhibits 50% of cytopathic effect.

**VLP expression plasmids.** For pGag, the cDNA sequence encoding the Gag capsid protein of the MLV Gag (Uniprot: PODOG8.1) without its C-terminal Pol sequence was obtained by enzymatic digestion from plasmid pBL36-HCV and cloned into the phCMV expression vector. For SARS-CoV-2 spike protein variants, the cDNA sequences were also cloned in the phCMV expression vector. All plasmids were verified by sequencing.

**VLP generation, production, purification and validation.** VLPs were produced by transient transfection of either HEK293 cells or HEK293-1267 cells, with pGag, pGag-N₁, pGag-N₂, pS and its variants, and pM plasmid DNA using PEI as transfection reagent. Cells were transfected at 70% confluence in T175 flasks with 70 µg of total DNA per flask at a PEI: DNA mass ratio of 3:1. VLP-containing supernatants were harvested 72 h post-transfection, filtered through a 0.45 µm-pore size membrane, and concentrated 20 x in a centrifugal filter device (Centricon^{®} Plus-70-100 K, Millipore) and purified by ultracentrifugation through a 20% sucrose cushion in an SW41T Beckman rotor (25,000 rpm, 4 h, at 4 °C). Pellets were resuspended in sterile TNE buffer (50mM Tris-HCl pH 7.4, 100mM NaCl, 0.1mM EDTA). Proteins were measured using the Bradford method. For western blotting, proteins were resolved by 10% SDS-PAGE and transferred onto PVDF membranes before incubation with specific primary antibodies. Alkaline phosphatase-conjugated secondary antibodies were used and they were detected by BCIP/NBT substrate.

**Immunizations.** Golden Syrian Hamsters were fasted 4 h and then orally immunized with two weekly doses of 100 µg of different VLPs. For subcutaneous immunization, two weekly doses of 10 µg of different VLPs were administered. Animals from the negative control group (naive) received oral immunizations with vehicle alone. Animals were not anesthetized during immunizations.

**Fluid collection.** Blood was collected weekly from the retro-orbital sinus of hamsters and serum was separated and stored at -80 °C. Bronchoalveolar lavage (BAL) was collected through the trachea by injection-aspiration of 1 mL PBS with protease inhibitors.

**Enzyme-linked immunosorbent assay (ELISA) tests.** The levels of IgG and IgA antibodies against spike protein were determined by ELISA by sensitized the plate with homogenates of killed whole virus produced *in vitro.* Quantification of Spike was performed using purified proteins: Human coronavirus HCoV-229E Spike Protein (S1+S2 ECD) (Sino Biological, Inc. 40605-V08B) and SARS-CoV-2 Spike Protein (Active Trimer) R&D Cat. # 10549-CV). The following secondary antibodies were used: Mouse anti-Hamster IgG Cocktail, Clone: G94-56, G70-204 (BD Biosciences, Cat. # 554009). Mouse anti-Hamster IgM, Clone: G188-9, (BD Biosciences Cat. # 554035). Hamster Immunoglobulin A (IgA) ELISA Kit (MyBiosources Cat. # MBS029668). Mouse monoclonal (H6) anti-SARS-CoV-2 spike glycoprotein (Abcam Cat. # ab273169).

**Statistical analyses.** For *in vitro* proteolytic densitometry experiments, two-tailed unpaired Student's t-test was used. Prism (GraphPad Software) was used to perform one-way or two-way ANOVA on datasets with Tukey's multiple comparisons test or Bonferroni post-test, respectively. All figures show mean ± s.e.m. Statistically significant differences are indicated in each graph as ^{∗}*p*< 0.05, ^{∗∗}*p*< 0.01 and ^{∗∗∗}*p*< 0.001 and ns = not significant.

### Example I - Immunogenicity of different variants of S in mice

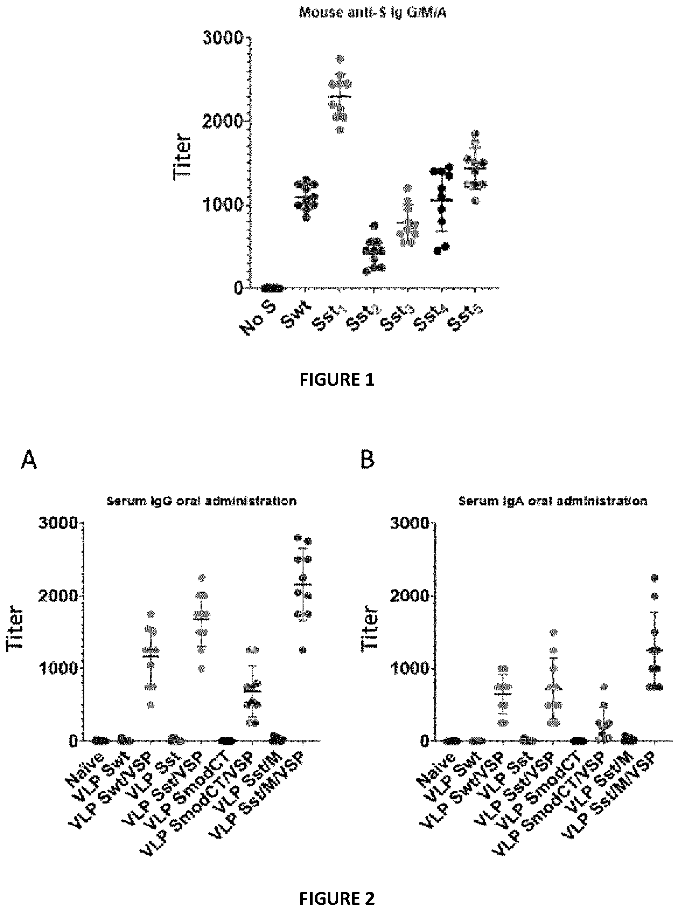

The experiments were conducted with the Spike protein (S) of SARS-CoV-2. Several variants of the Spike protein (or subtypes referred to as "Sst") for stabilization of the receptor-binding domain (RBD) in the open conformation and S protein stabilized in the pre-fusion state were designed. Several point mutations were used: Cys-molecular clamps, Furin-cleavage site elimination and Pro substitutions. Then, the VLPs were produced as described and validated for the correct composition. e-VLPs were orally administered to Balb/c mice and the level of serum and BAL Igs was determined by ELISA (**Figure 1**).

Notably, Spike variants lacking the Furin-cleaved site (modFurinCS) and with the two Pro substitutions were the most efficient in eliciting a high level of antibodies. Identical experiments performed in Ob/Ob, Db/Db and aged mice of both sexes showed similar results (not shown), indicating that regardless of underlying condition, sex or age of the mice, oral administration of VSP-e-VLPs does not vary between groups. Therefore, the most effective variant of S, i.e. Sst1, was selected for inclusion on the e-VLPs for immunizations and challenge experiments in the Golden Syrian Hamster model.

### Example II - Antibody responses to oral administration of different vaccine formulations

It is known that the C-terminal cytoplasmic tail (CT) of the SARS-CoV-2 S is important for proper glycosylation of the S glycoprotein. It was thus hypothesized that because there are numerous glycosylated sites near the receptor binding domain (RDB), differential glycosylation of S should influence the generation of efficient Nab. For this reason, the CT was modified to abolish endoplasmic reticulum (ER) retention (modCT). Moreover, the envelope membrane protein M of SARS-CoV-2 was incorporated into the e-VLPs. Indeed, M is known to retain S at the ER for improvement of the first steps of glycosylation and, subsequently, remains attached to the CT of S during their journey throughout the Golgi apparatus, where final glycosylation is accomplished. Although that was the main reason for including M in the e-VLPs, subsequent reports showed a specific T cell response to several epitopes of this protein in patients recovered after suffering Covid-19. Thus, incorporation of M in the envelope of the e-VLPs could not only benefit proper glycosylation of S but also the production of a stronger cellular response to the virus. Therefore, e-VSPs with/without M and with/without a modified CT were generated. e-VLPs with the incorporation onto the VLP surface of a VSP derived from Giardia, for oral administration, and others without VSPs, for i.m. injection, were also produced in order to compare both immunization routes.

Oral administered formulations showed that the absence of the *Giardia* VSP decorating different e-VLPs avoided any immune response, most likely due to destruction of the VLPs along the upper small intestine **(****Figures 2A** and **2B****).** Additionally, the modification of the CT of S appeared detrimental in inducing either serum IgG or IgA generated in the animals as compared with S having the wild type CT. However, IgG and IgA serum antibody titers augmented when M was incorporated into the VSP-e-VLPs.

### Example III - Antibody responses to intramuscular administration of different vaccine formulations

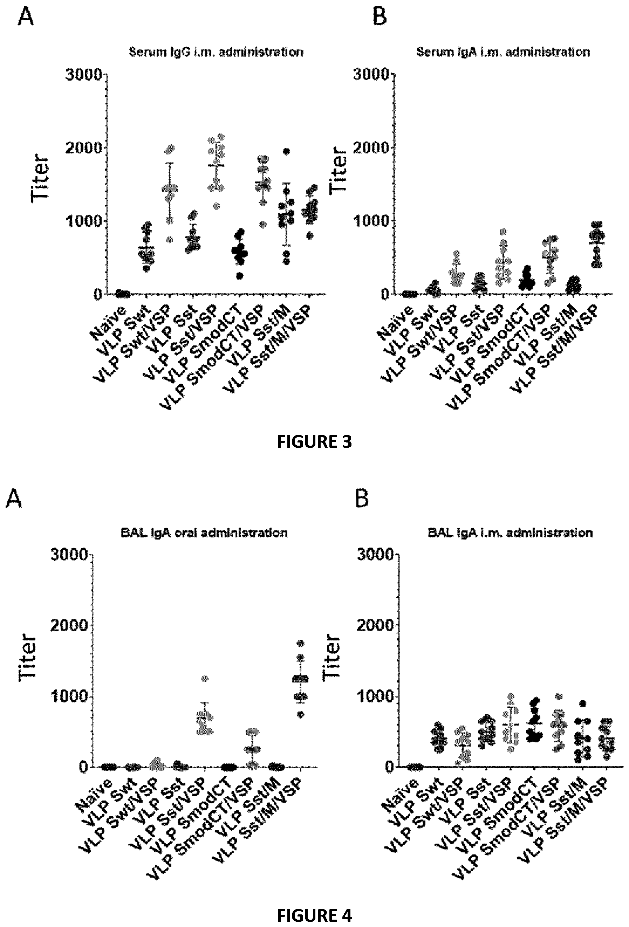

VSP-e-VLPs administered intramuscularly (i.m.) to hamsters induced high levels of both Igs in serum and the presence of the *Giardia* VSP on the e-VLPs promoted higher levels of antibodies than the plain e-VLP **(****Figure 3****).** The levels of serum IgG were higher than those of IgA, as expected. In all the i.m.-administered formulations, important variations were found among individual animals. However, these values were acceptable if compared to those obtained by other vaccine formulations or in plasma obtained from convalescent patients.

The differences observed between the same formulations administered either orally or intramuscularly in these animals suggest that although the oral route is expected to show higher degree of variation along animals, it was not as variable between the different formulations, which could be explained by the type of generated Igs. Notably, considering the i.m. administration was done in the absence of any added adjuvant, the high immunogenicity of VSP-e-VLPs can be explained by the adjuvant properties of the VSPs, which have been demonstrated to activate TLR-4. The lower immunogenicity of the e-VLPs lacking VSPs may only rely on the particulate nature of VLPs and the repetitive exposure of the antigen on their surface.

These results clearly show that the presence of the VSP on the surface of the e-VLP is essential for avoiding destruction of the particles when administered orally but plain e-VSP generate a significant response when administered by injection in the absence of adjuvant, response that strongly increased when the VSP was present.

### Example IV - Bronchoalveolar lavage IgA responses

When the presence of IgA was analyzed in animals immunized orally or by injection with those formulations, it was noticed that intramuscularly immunized animals have a consistent low level of IgA titers as compared with those administered orally, in which only the e-VLP version containing VSP, M, and stabilized S showed high titers of IgA in bronchoalveolar lavages **(****Figure 4****).** Again, these results confirm the high immunogenicity of e-VLP formulations and that VSPs are crucial to protect and adjuvant the particles for oral administration.

### Example V - Neutralizing antibodies against SARS-CoV-2 entry

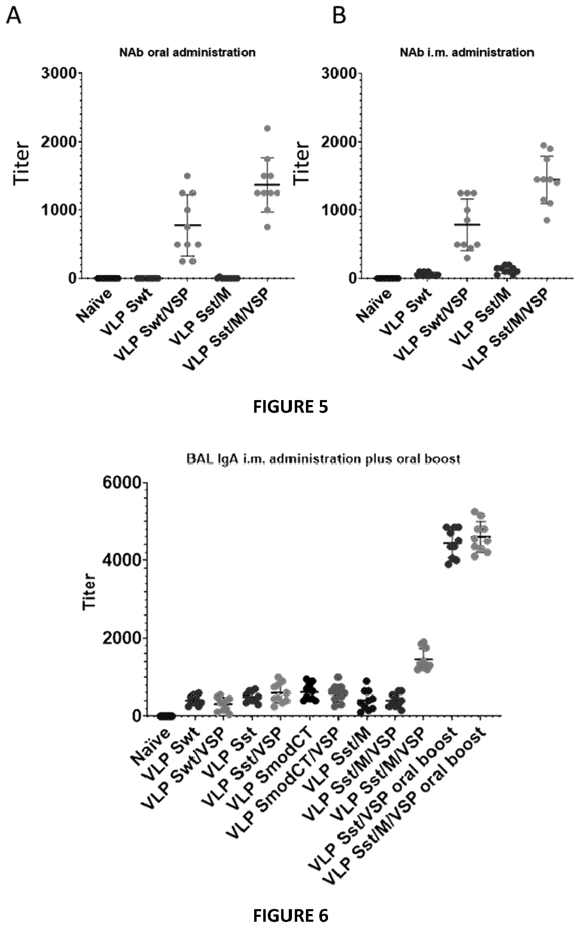

Additionally, IgA present in both orally and intramuscularly administered selected e-VLPs showed a high level of Nab in BAL, being those levels augmented when VSP and M were included into the particles **(****Figure 5****).** Remarkably, both wild-type and stabilized S proteins were able to generate comparable titers of Nabs, a phenomenon already observed along the different commercial vaccines already being administered to humans.

### Example VI - Oral boost after i.m. administration

Since VSP-e-VLPs are thermostable and even support several cycles of freeze/thawing, they are highly suitable for use in areas of the world where refrigeration supplies for vaccine injection are scarce. Indeed, despite that most countries have already started extensive vaccination campaigns, infections due to SARS-CoV-2 persist in most areas of the world due to the complications caused by the high levels of refrigeration of current vaccines, the lack of sterilizing immunity of vaccines administered intramuscularly or the need of trained personal for intramuscular administration.

For those reasons, an oral boost was applied to the animals previously vaccinated by injection. In those animals, a third dose of the formulation containing VSP, stabilized S and M induced a 6-fold increase in the levels of IgA in BAL as compared to those that only received two doses intramuscularly **(****Figure 6****).**

### Example VII - SARS-CoV-2 challenge

Finally, animals immunized with selected VSP-e-VLP formulations were challenged with SARS-CoV-2 and the response of the hamsters was determined. In this case, only formulations of e-VLPs in which stabilized S and M were present onto the particles with or without VSP pseudotyping were tested.

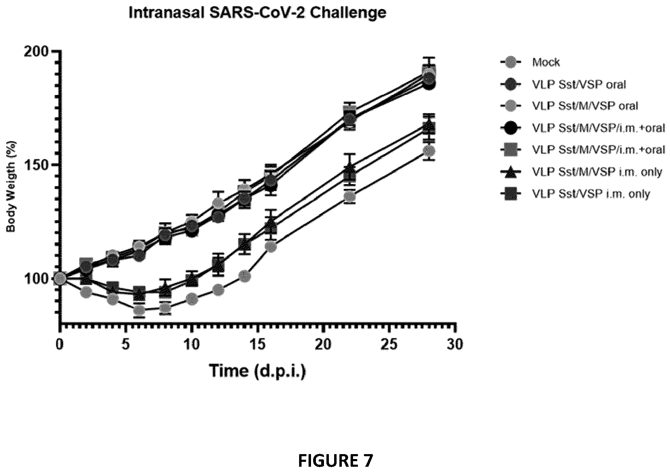

Results showed that oral immunization with VSP-e-VLPs prevented animal weight loss during the two weeks after infection similarly to those animals that were immunized by injection and then boosted orally. Conversely, controls animals lost weight during that period and then quickly recovered, as reported for hamster's experimental infections. An intermediate situation could be observed in the animals that were only immunized intramuscularly **(****Figure 7****).**

### VIII - Conclusions

These experimental results first show that it is possible to co-express SARS-CoV-2 envelope proteins together with *Giardia* VSPs on e-VLPs such as to generate NAb against SARS-CoV-2 after oral administration. While plain e-VLPs did not generate any Ab responses, VSP-e-VLPs generated Ab responses in the range of the response to i.m. administration. This is remarkable as it indicates that the VSPs protect SARS-CoV-2 Env proteins from degradation while preserving their proper conformation.

Moreover, these results also show the dual properties of VSPs, which not only afford protection from degradation, but also have a potent adjuvant effect. Indeed, when vaccines are administered i.m., VSP-e-VLP always led to higher titers of antibodies than their plain e-VLPs. Of note, a SARS-CoV-2 VLP was recently reported to generate good Nab response after i.m. administration, but with no reports of IgA at mucosal sites.

Besides its ease, oral administration is known for also having the advantage of triggering better mucosal immunity. This is indeed the case here, with high levels of plasma but also BAL IgA detectable only after oral administration. This is an obvious advantage for a vaccine against SARS-CoV-2 as it should reduce viral transmission. In this line, SARS-CoV-2 was still detected in BAL of vaccinated macaques that otherwise appeared protected from infection.

The specific T cells response was not tested in this study. However, it is known that e-VLP do induce robust such response; indeed, using VSP-HA-VLP, strong CTLs that were able to kill HA-expressing tumor cells (Serradell et al., Nat. Commun. 10:361 (2019)). Moreover, the IgG and IgA response here are notoriously T cell dependent and the good antibody response thus attest a good T cell response.

In this line, it was previously shown that the fusion of a viral peptide to GAG, the retroviral protein precursor that drives the formation and release of the viral particle/VLP, allows producing additional strong T cell responses against this peptide. The fusion to Gag of large fragments or the SARS-CoV-2 N structural protein, or a stretch of immunodominant peptides would clearly be a mean to further enhance the immunogenicity of VSP-e-VLP.

SARS-CoV-2 VSP-e-VLP could be used as a stand-alone vaccine, likely with a prime-boost scheme of administration. Since VSP-e-VLPs are thermostable, retaining their properties at room temperatures, they could be particularly advantageous for vaccination in countries that would benefit from a thermostable vaccine. VSP-e-VLPs could also be used as a boost for other vaccine designs.

In this line, the duration of the protection afforded by the currently used vaccines remains unknown. The follow-up of infected patients indicate that, at least for some patients, the persistence of the Nab and duration of the protection might be of a few months. These, plus the advent of viral variants, make it likely that it will be necessary to boost the immune response of vaccinees regularly. For some vaccine design, and particularly those based on adenoviral vectors, the re-administration of the same vector might not be very efficient due to the immune response generated against the vector. For these reasons, a boost with VSP-e-VLPs might be particularly interesting. For other vaccine designs, and especially if repeated administration are needed other years, an orally administered vaccine might be more acceptable.

Altogether, given each vaccine design specific issues (thermostability, side effects, lack of mucosal immunity induction, immunogenicity against the vector, among other drawbacks), the availability of multiple vaccines against SARS-CoV-2 is the guarantee of having more chances to control the pandemic.

Consequently, the thermostable orally administered e-VLP vaccine will be a valuable addition to the arsenal against this virus.

## Claims

1. A viral-like particle (VLP) displaying at its surface at least:
- a variable surface protein (VSP), a VSP-like protein or a fragment thereof of a microorganism selected in the group consisting of *Giardia, Tetrohymeno, Paramecium* and *Entamoeba* species, and
- a viral protein or a fragment thereof of a *Coronaviridae* virus.

2. The VLP according to claim 1, wherein the microorganism is selected from the group consisting of *Giardia lamblia, Tetrahymena thermophila, Paramecium tetraurelia, Entamoeba histolytica.*

3. The VLP according to claims 1 or 2, wherein the VSP, VSP-like protein or fragment thereof, preferably the extracellular region of the VSP, is fused with the transmembrane region of the vesicular stomatitis virus G protein (VSV-G).

4. The VLP according to any one of claims 1 to 3, wherein the *Coronaviridae* virus is a coronavirus, preferably selected from the group consisting of SARS-CoV-1, SARS-CoV-2, MERS-CoV and variants thereof.

5. The VLP according to any one of claims 1 to 4, wherein the viral protein is selected from the group consisting of Spike (S) protein, membrane (M) protein, envelope (E) protein and nucleocapsid (N) protein, preferably S protein.

6. The VLP according to any one of claims 1 to 5, wherein the VLP displays at its surface:
- a VSP, a VSP-like protein or a fragment thereof of *Giardia lamblia,* and
- a S protein or a fragment thereof of SARS-CoV-2.

7. The VLP according to any one of claims 1 to 6, wherein the VLP further comprises a Gag protein fused to its carboxy terminus to the N protein or a fragment thereof from a *Coronaviridae* virus, preferably from SARS-CoV-2.

8. The VLP according to any one of claims 1 to 7, wherein S protein is stabilized in pre-fusion.

9. The VLP according to any one of claims 1 to 8, wherein the VLP displays at its surface at least two distinct viral proteins, preferably S and M proteins.

10. A VLP as defined in any one of claims 1 to 9, for use as a medicament.

11. A VLP as defined in any one of claims 1 to 9, for use in the treatment or prevention of an infection by a virus from the *Coronaviridae* family.

12. A VLP as defined in any one of claims 1 to 9, for use as a vaccine against an infection by a virus from the *Coronaviridae* family.

13. The VLP for use as defined in any one of claims 10 to 12, said VLP being orally or intranasally administered to a patient.

14. The VLP for use according to any one of claims 10 to 13, wherein the VLP is administered as a boost to a patient who has previously received a vaccine by subcutaneous or intramuscular injection, against the virus from the *Coronaviridae* family.

15. A pharmaceutical composition comprising a VLP as defined in any one of claims 1 to 9.
